# EUROPÄISCHE PATENTANMELDUNG

(11) **EP 2 468 224 A1**
(43) Veröffentlichungstag der Anmeldung: **27.06.2012**
(21) Anmeldenummer: 10016024.1
(22) Anmeldetag: 23.12.2010
(51) Int. Cl.: A61F 9/008, A61F 9/01, B23K 26/00, B23K 26/40, B23K 26/06

(54) **Vorrichtung zur Bearbeitung von Augengewebe**

(71) Anmelder: Ziemer Ophthalmic Systems AG, 2562 Port (CH)
(72) Erfinder: Rathjen, Christian, 28197 Bremen (DE)
(74) Vertreter: Rentsch Partner AG

(57) **Zusammenfassung**

Eine ophthalmologische Vorrichtung (1) zur Bearbeitung von Augengewebe, mit einem Femtosekundenlaseroszillator (11) zur Erzeugung von Femtosekundenlaserpulsen (FP) und einem Lichtprojektor (16) zur fokussierten Projektion der Laserpulse (P) auf oder in das Augengewebe ist überdies mit einem Picosekundenlasermodul (12) zur Erzeugung von Picosekundenlaserpulsen (PP) versehen. Dabei können dem Lichtprojektor (16) zur Bearbeitung des Augengewebes wahlweise Femtosekundenlaserpulse (FP) und/oder Picosekundenlaserpulse (PP) zugeführt werden. Die ophthalmologische Vorrichtung (1) ist somit wahlweise zur Ausführung von präzisen Schnitten im Augengewebe mittels der Femtosekundenlaserpulse (FP) und zur Fragmentierung von Augengewebe durch Gewebezertrümmerung mittels der Picosekundenlaserpulse (PP) einsetzbar.

## Beschreibung

### Technisches Gebiet

Die vorliegende Erfindung betrifft eine ophthalmologische Vorrichtung zur Bearbeitung von Augengewebe. Die vorliegende Erfindung betrifft insbesondere eine ophthalmologische Vorrichtung zur Bearbeitung von Augengewebe mit einem Femtosekundenlaseroszillator zur Erzeugung von Femtosekundenlaserpulsen und einem Lichtprojektor zur fokussierten Projektion der Laserpulse auf oder in das Augengewebe.

### Stand der Technik

Die Lasertechnologie wird seit Jahrzehnten für den universellen Einsatz zum Bearbeiten von unterschiedlichsten Materialien propagiert. Im Bereich der Ophthalmologie werden Laser zur Bearbeitung von Augengewebe, insbesondere zum Abtragen, Schneiden und Fragmentieren (Zertrümmern) von Augengewebe eingesetzt. Für präzise Schnitte im Augengewebe, insbesondere in der Cornea, beispielsweise zum Schneiden eines Cornealappens im Rahmen einer laserbasierten in-situ Keratomileusis (LASIK) für die refraktive Korrektur eine Auges, werden bevorzugt stark fokussierte Femtosekundenlaserpulse eingesetzt, die Pulsbreiten (d.h. zeitliche Pulslänge oder Pulsdauer) von typisch 100fs bis 1000fs (1fs=10⁻¹⁵s) aufweisen. Ein Femtolasersystem zum Schneiden von Cornealappen wird beispielsweise in EP 1 731 120 beschrieben. Die Femtosekundenlaserpulse werden in einer Femtosekundenstrahlquelle mit einem Femtosekundenlaseroszillator erzeugt, der in der Regel mit Pulsraten im MHz-Bereich arbeitet. Femtosekundenstrahlquellen, die als reine Laseroszillatoren, ohne nachgeschaltete optische Verstärker ausgeführt sind, sind im Vergleich zu Femtosekundenstrahlquellen in sogenannter MOPA-Konfiguration (Master Oscillator Power Amplifier) mit nachgeschaltetem optischem Verstärker, stabiler, kompakter und kostengünstiger. Femtosekundenlaseroszillatoren ohne nachgeschaltete optische Verstärker erzeugen jedoch Femtosekundenlaserpulse mit bedeutend geringerer Pulsenergie als Femtosekundenstrahlquellen in MOPA-Konfiguration. Die begrenzte Pulsenergie eines reinen Femtosekundenlaseroszillators reicht beispielsweise nicht aus, um Augengewebe, beispielsweise das Gewebe einer Augenlinse, mittels Gewebezertrümmerung zu Fragmentieren.

### Darstellung der Erfindung

Es ist eine Aufgabe der vorliegenden Erfindung eine ophthalmologische Vorrichtung zur Bearbeitung von Augengewebe vorzuschlagen, welche zumindest einige Nachteile der bekannten Systeme nicht aufweist. Es ist insbesondere eine Aufgabe der vorliegenden Erfindung eine ophthalmologische Vorrichtung zur Bearbeitung von Augengewebe mit einem Femtosekundenlaseroszillator vorzuschlagen, welche sowohl zum Ausführen von präzisen, gewebeverträglichen Schnitten im Augengewebe als auch zum Fragmentieren von Augengewebe geeignet ist.

Gemäss der vorliegenden Erfindung werden diese Ziele durch die Merkmale der unabhängigen Ansprüche erreicht. Weitere vorteilhafte Ausführungsformen gehen ausserdem aus den abhängigen Ansprüchen und der Beschreibung hervor.

Die oben genannten Ziele werden durch die vorliegende Erfindung insbesondere dadurch erreicht, dass eine ophthalmologische Vorrichtung zur Bearbeitung von Augengewebe, welche einen Laserpulsgenerator zum Erzeugen von Laserpulsen mit einem Femtosekundenlaseroszillator zur Erzeugung von Femtosekundenlaserpulsen, und einen Lichtprojektor zur fokussierten Projektion der Laserpulse auf oder in das Augengewebe umfasst, überdies ein Picosekundenlasermodul zur Erzeugung von Picosekundenlaserpulsen umfasst, wobei dem Lichtprojektor zur Bearbeitung des Augengewebes wahlweise Femtosekundenlaserpulse und/oder Picosekundenlaserpulse zuführbar sind.

Mittels der Femtosekundenlaserpulse können im Augengewebe präzise Schnitte ausgeführt werden, beispielsweise in der Cornea, dabei werden die Femtosekundenlaserpulse mit einer Pulsenergie von 10nJ bis 1000nJ und einer Pulsrate im Bereich von 1-1 00MHz fokussiert ins Gewebe projiziert, wobei im Wesentlichen sich nur geringste Pulsenergien in das an die Schnittfläche angrenzende Gewebe ausbreiten und somit keine unerwünschte Gewebebeeinträchtigung stattfindet. Mittels der Picosekundenlaserpulse kann das Augengewebe durch Gewebezertrümmerung fragmentiert werden, beispielsweise in der Augenlinse, dabei werden die Picosekundenlaserpulse mit einer vergleichsweise höheren Pulsenergie von 1µJ bis 100µJ und einer vergleichweise tieferen Pulsrate im Bereich von 1 KHz-1 MHz fokussiert ins Gewebe projiziert. Im Gegensatz zu Femtosekundenlaserstrahlquellen sind Picosekundenlaserstrahlquellen sehr robust und preiswert bei hohen und mittleren Leistungen. Die im Vergleich zu Femtosekundenlaserpulsen zur Gewebewechselwirkung höheren benötigten Pulsenergien bei Picosekundenlaserpulsen und die damit einhergehende deutlich geringere Gewebeverträglichkeit spielen bei Anwendungen wie der Fragmentierung keine Rolle, da das fragmentierte respektive zertrümmerte Gewebe nachträglich entfernt wird. Ganz im Gegenteil, die höheren Pulsenergien sind sogar gewünscht, da damit eine effektivere Gewebezertrümmerung erreicht werden kann.

In einer bevorzugten Ausführungsvariante umfasst die Vorrichtung ein Steuermodul, das eingerichtet ist, die Vorrichtung wahlweise in mindestens einen der nachfolgenden Betriebsmodi zu setzen und zu steuern:
- ein erster Betriebsmodus, in welchem vom Laserpulsgenerator dem Lichtprojektor Femtosekundenlaserpulse zum Schneiden des Augengewebes zugeführt werden,
- ein zweiter Betriebsmodus, in welchem vom Laserpulsgenerator dem Lichtprojektor Picosekundenlaserpulse zum Fragmentieren des Augengewebes zugeführt werden,
- ein dritter Betriebsmodus, in welchem vom Laserpulsgenerator dem Lichtprojektor zur Bearbeitung des Augengewebes jeweils mit zeitlich versetztem Pulsbeginn ein Picosekundenlaserpuls gefolgt von einem Femtosekundenlaserpuls zugeführt werden, und
- ein vierter Betriebsmodus, in welchem vom Laserpulsgenerator dem Lichtprojektor zur Bearbeitung des Augengewebes jeweils mit zeitlich versetztem Pulsbeginn ein Femtosekundenlaserpuls gefolgt von einem Picosekundenlaserpuls zugeführt werden.

Durch die Kombination von zwei nacheinander folgenden und sich beispielsweise teilweise überlappenden Laserpulsen im dritten und vierten Betriebsmodus kann die Pulsenergie der beiden Pulse geeignet kombiniert werden. Damit lassen sich neue Wechselwirkungsmechanismen ausnutzen, die so nicht mit der ausschliesslichen Verwendung von Femto- oder Picosekundenlaserpulsen zugänglich sind. Beispielsweise kann mit einem vorangehenden Femtosekundenlaserpuls ein initiale Zündung bewirkt und mit dem nachfolgenden Picosekundenlaserpuls zusätzliche Energie in die gezündete Wechselwirkungszone gepumpt werden. Umgekehrt kann z.B. mit Picosekundenlaserpulsen unterhalb der optischen Durchbruchschwelle das Gewebe in einen aktivierten Zustand versetzt werden, der tiefere Pulsenergien der zum Schneiden verwendeten Femtosekundenlaserpulse ermöglicht, was wiederum präzisere Schnitte erlaubt.

In einer Ausführungsvariante umfasst das Picosekundenlasermodul einen Picosekundenlaseroszillator zum Erzeugen der Picosekundenlaserpulse.

In einer anderen Ausführungsvariante umfasst das Picosekundenlasermodul einen Pulsstrecker, welcher eingerichtet ist, Femtosekundenlaserpulse vom Femtosekundenlaseroszillator in Picosekundenlaserpulse zu strecken.

In einer weiteren Ausführungsvariante umfasst das Picosekundenlasermodul ein im Femtosekundenlaseroszillator angeordnetes einschaltbares Verstimmglied, welches eingerichtet ist, den Femtosekundenlaseroszillator im eingeschalteten Zustand so zu verändern, dass der Femtosekundenlaseroszillator als Picosekundenlaseroszillator betreibbar ist. Im eingeschalteten Zustand kann das Verstimmglied beispielsweise die spektrale Bandbreite des Femtosekundenlaseroszillators so beschränken, dass der Femtosekundenlaseroszillator als Picosekundenlaseroszillator arbeitet und Picosekundenlaserpulse erzeugt.

In einer Ausführungsvariante ist eine Polarisationsmodulation vorgesehen, derart, dass die Femtosekundenlaserpulse und die Picosekundenlaserpulse unterschiedlich, beispielsweise normal zueinander, polarisiert sind, und dass die Vorrichtung einen Polasierungsstrahlteiler umfasst, um dem Lichtprojektor wahlweise Femtosekundenlaserpulse oder Picosekundenlaserpulse zuzuführen. Zum Beispiel ist dem Femtosekundenlaseroszillator und/oder dem Picosekundenlasermodul ein Polarisationsmodulator nachgeschaltet, oder der Femtosekundenlaseroszillator und/oder das Picosekundenlasermodul sind eingerichtet die Femtosekundenlaserpulse respektive die Picosekundenlaserpulse unterschiedlich zu polarisieren.

In einer weiteren Ausführungsvariante ist zum wahlweisen Zuführen von Femtosekundenlaserpulsen respektive Picosekundenlaserpulsen zum Lichtprojektor mindestens ein steuerbarer Shutter, ein steuerbarer Drehspiegel, ein elektromechanischer Schalter und/oder ein elektronischer Schalter vorgesehen.

Vorzugsweise sind die Femtosekundenlaserpulse zum Schneiden des Augengewebes fokussiert in die Cornea projizierbar, und die Picosekundenlaserpulse sind zum Fragmentieren des Augengewebes fokussiert in die Augenlinse projizierbar.

Vorzugsweise ist der Laserpulsgenerator mit dem Femtosekundenlaseroszillator und dem Picosekundenlasermodul in einem gemeinsamen Gehäuse angeordnet, und die ophthalmologische Vorrichtung umfasst zum Thermalisieren des Laserpulsgenerators eine mit dem Gehäuse verbundene Kühlvorrichtung. Somit können sich der Femtosekundenlaseroszillator und das Picosekundenlasermodul die aufwendige und kostspielige Kühlvorrichtung zum Thermalisieren teilen.

Vorzugsweise umfasst die Vorrichtung ein mit dem Femtosekundenlaseroszillator und dem Picosekundenlasermodul verbundenes gemeinsames elektronisches Lasersteuermodul zum Steuern des Femtosekundenlaseroszillators und des Picosekundenlasermoduls, und/oder ein mit dem Femtosekundenlaseroszillator und dem Picosekundenlasermodul verbundenes gemeinsames Speisegerät zur elektrischen Speisung des Femtosekundenlaseroszillators und des Picosekundenlasermoduls. Somit können sich der Femtosekundenlaseroszillator und das Picosekundenlasermodul das Lasersteuermodul und/oder das Speisegerät teilen.

In einer Ausführungsvariante ist dem Femtosekundenlaseroszillator ein Pulsselektor zum Erzeugen einer bestimmten Pulsrate nachgeschaltet, und die Pulsrate ist beispielsweise abhängig von einem gewählten Betriebsmodus setzbar.

In einer weiteren Ausführungsvariante umfasst die Vorrichtung eine gemeinsame Pumpquelle für den Femtosekundenlaseroszillator und das Picosekundenlasermodul.

In einer Ausführungsvariante umfasst das Picosekundenlasermodul einen optischen Verstärker.

In einer weiteren Ausführungsvariante umfasst das Picosekundenlasermodul einen optischen Kompressor zum Komprimieren der Pulslänge der Picosekundenlaserpulse.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:
- Figur 1:: zeigt ein Blockdiagramm, welches schematisch eine ophthalmologische Vorrichtung mit einem Laserpulsgenerator illustriert, welche einem Lichtprojektor für die Bearbeitung von Augengewebe wahlweise Femtosekundenlaserpulse und/oder Picosekundenlaserpulse zur fokussierten Projektion zuführt.
- Figur 2:: zeigt ein Blockdiagramm, welches schematisch eine Ausführung der ophthalmologischen Vorrichtung illustriert, in welcher der Laserpulsgenerator einen Femtosekundenlaseroszillator und einen Picosekundenlaseroszillator umfasst.
- Figur 3:: zeigt ein Blockdiagramm, welches schematisch eine Ausführung der ophthalmologischen Vorrichtung illustriert, in welcher Femtosekundenlaserpulse und Picosekundenlaserpulse über Polarisationsmodule und einen Polasierungsstrahlteiler dem Lichtprojektor zugeführt werden.
- Figur 4:: zeigt ein Blockdiagramm, welches schematisch eine Ausführung der ophthalmologischen Vorrichtung illustriert, in welcher der Laserpulsgenerator einen Femtosekundenlaseroszillator und einen Pulsstrecker zur Erzeugung der Picosekundenlaserpulse umfasst.
- Figur 5:: zeigt ein Blockdiagramm, welches schematisch eine Ausführung der ophthalmologischen Vorrichtung illustriert, in welcher der Laserpulsgenerator einen Femtosekundenlaseroszillator und ein darin angeordnetes Verstimmglied zur Erzeugung der Picosekundenlaserpulse umfasst.
- Figuren 6a-6b:: illustrieren schematisch, von oben nach unten, einen Femtosekundenlaserpuls, eine Streckung des Femtosekundenlaserpulses, eine optische Verstärkung des gestreckten Pulses, und eine Komprimierung des verstärkten und gestreckten Pulses.

### Wege zur Ausführung der Erfindung

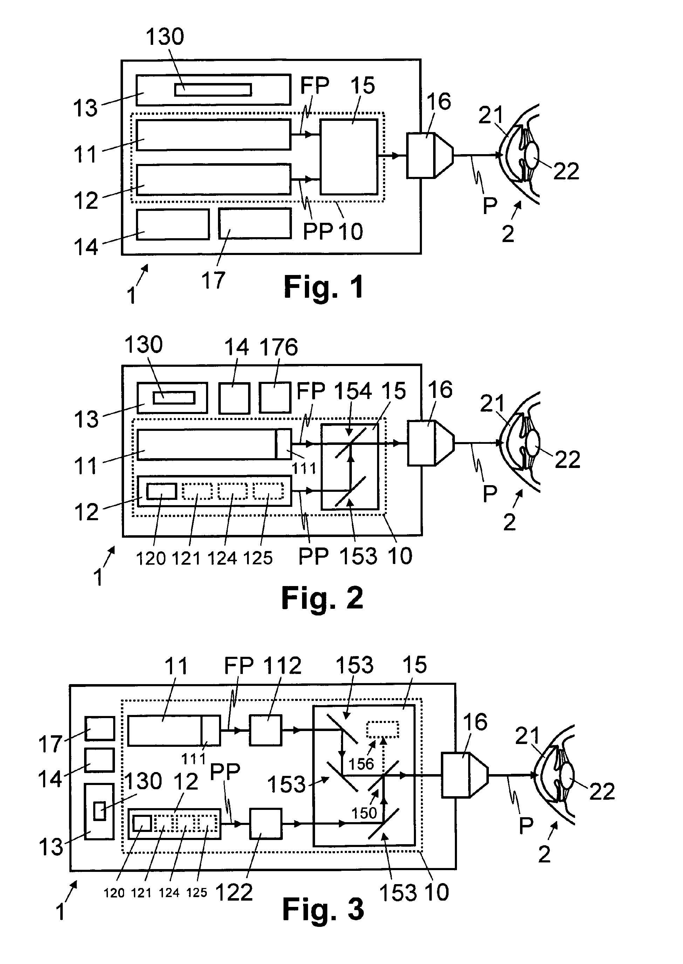

In den Figuren 1 bis 5 bezieht sich das Bezugszeichen 1 auf eine ophthalmologische Vorrichtung für die Bearbeitung von Gewebe eines Auges 2, insbesondere von Augengewebe der Cornea 21 und der Augenlinse 22. Die ophthalmologische Vorrichtung 1 umfasst einen Laserpulsgenerator 10 zum Erzeugen von Laserpulsen P und einen Lichtprojektor 16 zur fokussierten Projektion der Laserpulse P auf oder in das Augengewebe, insbesondere in oder auf die Cornea 21 und/oder die Augenlinse 22.

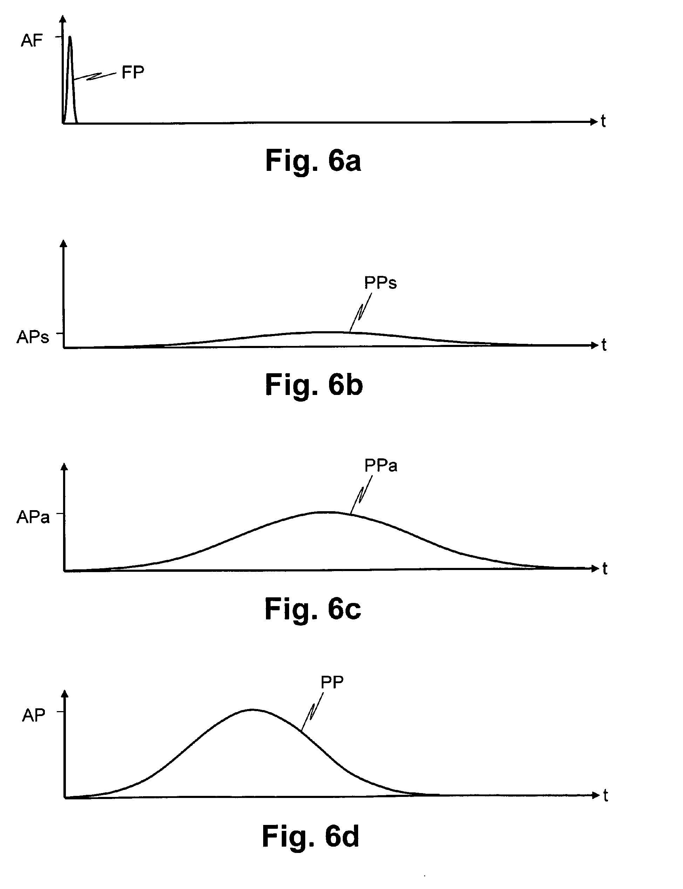

Wie in den Figuren 1 bis 5 schematisch dargestellt ist, umfasst der Laserpulsgenerator 10 einen Femtosekundenlaseroszillator 11 zur Erzeugung von Femtosekundenlaserpulsen FP. Figur 6a illustriert schematisch den zeitlichen Verlauf eines Femtosekundenlaserpulses FP mit einer Amplitude AF. Vorzugsweise aber nicht zwingend ist dem Femtosekundenlaseroszillator 11 ein Pulsselektor 111 nachgeschaltet, der eingerichtet ist, die Femtosekundenlaserpulse FP mit einer bestimmten Pulsrate weiterzuleiten.

Der Laserpulsgenerator 10 umfasst zudem ein Picosekundenlasermodul 12 zur Erzeugung von Picosekundenlaserpulsen PP. Picosekundenlaserpulse PP weisen eine Pulsbreite (zeitliche Pulslänge oder Pulsdauer) von typisch 5ps bis 1000ps (1ps=10⁻¹²s) auf. Nachfolgend werden mit Bezug auf die Figuren 2 bis 5 verschiedene Varianten zur Erzeugung der Picosekundenlaserpulse PP beschrieben.

In den Varianten gemäss den Figur 2 und 3 umfasst das Picosekundenlasermodul 12 einen Picosekundenlaseroszillator 120 zum Erzeugen der Picosekundenlaserpulse PP. Vorzugsweise umfasst die ophthalmologische Vorrichtung 1 eine gemeinsame Pumpquelle für den Femtosekundenlaseroszillator 11 und das Picosekundenlasermodul 12. Je nach Ausführung ist dem Picosekundenlaseroszillator 120 zudem ein Pulsselektor 121 nachgeschaltet, der eingerichtet ist, die Picosekundenlaserpulse PP mit einer bestimmten Pulsrate weiterzuleiten. Wie in den Figuren 2 und 3 schematisch dargestellt ist, sind dem Picosekundenlaseroszillator 120 zudem optional ein optischer Verstärker 124 zur Verstärkung der Picosekundenlaserpulse PP und/oder ein Kompressor 125 zum Komprimieren der Pulslänge der Picosekundenlaserpulse PP nachgeschaltet.

Der Fachmann wird verstehen, dass der Verstärker des Picosekundenlasermoduls 12 auch in der Form eines regenerativen Verstärkers, eines Multipass-Verstärkers oder als mehrstufiger Verstärker aufgebaut werden kann.

Der Fachmann wird überdies verstehen, dass in den Laseroszillatoren und Verstärkern Fasern und/oder Kristalle als aktive Medien verwendbar sind.

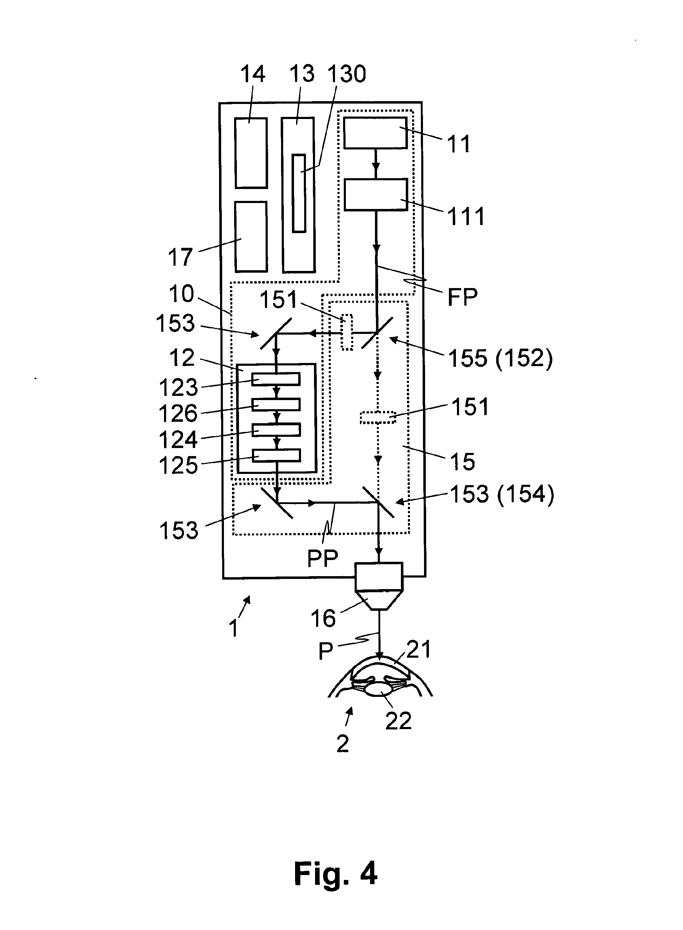

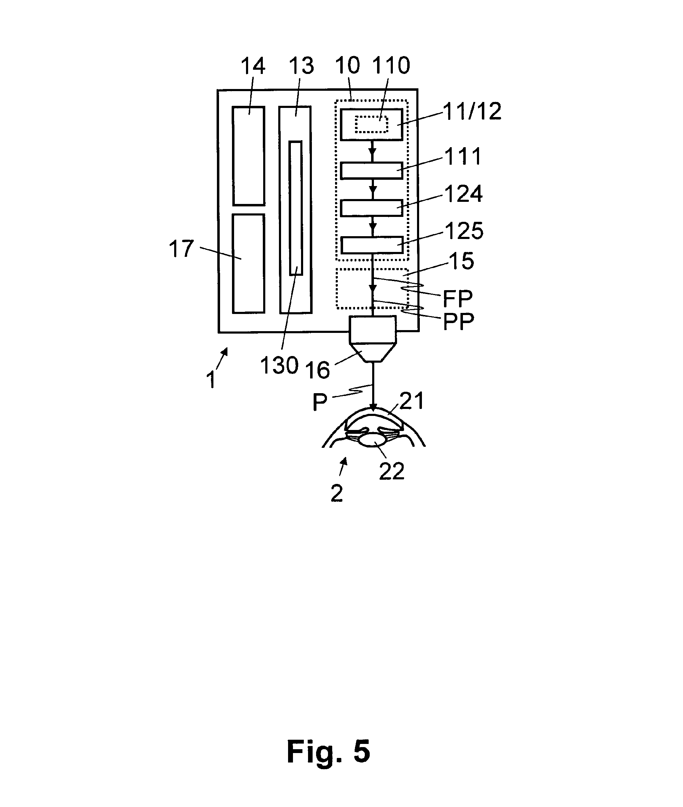

In den Varianten gemäss den Figur 4 und 5 umfasst das Picosekundenlasermodul 12 einen Pulswandler, welcher eingerichtet ist, die Picosekundenlaserpulse PP aus den vom Femtosekundenlaseroszillator 11 erzeugten Femtosekundenlaserpulsen FP zu generieren.

In der Variante gemäss Figur 4 ist der Pulswandler in Form eines im Picosekundenlasermodul 12 angeordneten Pulsstreckers 123 ausgeführt. Der Pulsstrecker 123 streckt die Femtosekundenlaserpulse FP auf eine Pulsbreite respektive Pulsdauer eines Picosekundenlaserpulses PP. Figur 6b illustriert schematisch einen sich aus der Streckung des in Figur 6a dargestellten Femtosekundenlaserpulses FP ergebenden Picosekundenlaserpuls PPs, wobei sich die Amplitude AF des Femtosekundenlaserpulses FP bei der Streckung auf die Amplitude APs des Picosekundenlaserpulses PPs abschwächt. Wie in der Figur 4 schematisch dargestellt ist, ist dem Pulsstrecker optional ein optischer Verstärker 124 zur Verstärkung der Picosekundenlaserpulse PP nachgeschaltet. Figur 6c illustriert schematisch einen sich aus der optischen Verstärkung des in Figur 6b dargestellten gestreckten Pulses PPs ergebenden verstärkten Picosekundenlaserpuls PPa mit der erhöhten Amplitude APa. Der Fachmann wird verstehen, dass abhängig von ihrer Leistung, Femtosekundenlaserpulse FP vor der Zuführung zum optischen Verstärker 124 des Picosekundenlasermodul in einem optischen Abschwächermodul abgeschwächt werden, um den optischen Verstärker 124 nicht zu überlasten. In einer Ausführungsvariante werden die Femtosekundenlaserpulse FP übermässig gestreckt, d.h. länger als die definierte Pulslänge der abzugebenden Picosekundenlaserpulse PP, wodurch die Femtosekundenlaserpulse FP so abgeschwächt werden, dass auf ein optisches Abschwächermodul verzichtet werden kann. Das Picosekundenlasermodul 12 umfasst dann entsprechend einen optischen Kompressor 125 zum Komprimieren der Pulslänge der übermässig gestreckten Femtosekundenlaserpulse PPs auf die definierte Pulslänge der abzugebenden Picosekundenlaserpulse PP. Figur 6d illustriert schematisch einen sich aus der Komprimierung des in Figur 6c dargestellten übermässig gestreckten Picosekundenlaserpulses PPs ergebenden Picosekundenlaserpuls PP mit weiter verstärkter Amplitude AP.

In der Variante gemäss Figur 5 ist der Pulswandler in Form eines im Femtosekundenlaseroszillator 11 angeordneten einschaltbaren Verstimmglieds 110 ausgeführt. Das Verstimmglied 110 ist eingerichtet, den Femtosekundenlaseroszillator 11 im eingeschalteten Zustand so zu verändern, dass der Femtosekundenlaseroszillator 11 als Picosekundenlaseroszillator 12 arbeitet und Picosekundenlaserpulse PP generiert. Das Verstimmglied 110 beschränkt beispielsweise die spektrale Bandbreite des Femtosekundenlaseroszillators 11 derart, dass dieser im eingeschalteten Zustand des Verstimmglieds 110 als Picosekundenlaseroszillator 12 betrieben wird. Das Verstimmglied 110 ist beispielsweise als Spektralfilter ausgeführt. Wie in der Figur 5 schematisch dargestellt ist, sind dem Femtosekundenlaseroszillator 11 optional ein Pulsselektor 111 zur Bestimmung der Pulsrate, ein optischer Verstärker 124 zur Verstärkung der Femtosekundenlaserpulse FP respektive Picosekundenlaserpulse PP und/oder ein Kompressor 125 zum Komprimieren der Pulslänge der Femtosekundenlaserpulse FP respektive Picosekundenlaserpulse PP nachgeschaltet.

Das Bezugszeichen 15 bezieht sich auf ein optisches Übertragungssystem zum Zuführen der vom Laserpulsgenerator 10 erzeugten Laserpulse zum Lichtprojektor 16. Je nach Ausführungsvariante umfasst das optische Übertragungssystem 15 Polasierungsstrahlteiler 150, Shutter 151, Strahlteiler 152, fixierte (Umlenk-)Spiegel 153, strahlvereinigende Elemente 154, z.B. teildurchlässige Spiegel, steuerbare Drehspiegel 155, und/oder Lichtleiter. Die Shutters 151 sind beispielsweise jeweils mit einem steuerbaren Drehspiegel in Kombination mit einem optischen Beam-Dump-Modul ausgeführt. Im durchlässigen, durchgeschalteten Zustand werden die Laserpulse vom Drehspiegel des Shutters 151 dem gewünschten Verbraucher zugeführt, beispielsweise dem Lichtprojektor 16 zur fokussierten Projektion in oder auf das Augengewebe. Im nichtdurchlässigen, gesperrten Zustand hingegen werden die Laserpulse vom Drehspiegel des Shutters 151 dem Beam-Dump-Modul zugeführt, wo sie absorbiert werden. In weiteren Varianten ist der Shutter elektro-optisch oder mechanisch, z.B. als Blende, ausgeführt.

In den Figuren 1 bis 5 bezieht sich das Bezugszeichen 17 auf ein gemeinsames Speisegerät zur elektrischen Speisung des Femtosekundenlaseroszillators 11 und des Picosekundenlasermoduls 12.

Der Femtosekundenlaseroszillator 11 und das Picosekundenlasermodul 12 des Laserpulsgenerators 10 sind in einem gemeinsamen Gehäuse angeordnet. Zum Thermalisieren des Laserpulsgenerators 10, d.h. des Femtosekundenlaseroszillators 11 und des Picosekundenlasermoduls 12, umfasst die ophthalmologische Vorrichtung 1 eine mit diesem Gehäuse verbundene Kühlvorrichtung 14. Das heisst der Femtosekundenlaseroszillator 11 und das Picosekundenlasermodul 12 werden durch eine gemeinsame Kühlvorrichtung 14 thermalisiert.

Weiterhin lassen sich bei der Verwendung eines gemeinsamen Gehäuses je nach Aufbau der einzelnen Strahlquellen optische Elemente (Pulsselektoren, z.B. Spiegel) gemeinsam nutzen.

Die wahlweise Zuführung der Femtosekundenlaserpulse FP und/oder Picosekundenlaserpulse PP zum Lichtprojektor 16 wird in verschiedenen Ausführungsvarianten unterschiedlich ausgeführt. In einigen Varianten erfolgt die wahlweise Zuführung der Femtosekundenlaserpulse FP und/oder Picosekundenlaserpulse PP zum Lichtprojektor 16 durch das optische Übertragungssystem 15.

In der Ausführungsvariante gemäss der Figur 2 erfolgt die Selektion von Femtosekundenlaserpulsen FP oder Picosekundenlaserpulsen PP durch entsprechendes Einund Ausschalten der Laseroszillatoren, d.h. durch Einschalten des Femtosekundenlaseroszillators 11 bei ausgeschaltetem Picosekundenlaseroszillator 120 respektive durch Einschalten des Picosekundenlaseroszillators 120 bei ausgeschaltetem Femtosekundenlaseroszillator 11. Zum Ein- und Ausschalten der Laseroszillatoren sind steuerbare elektromechanische Schalter und/oder elektronische Schalter vorgesehen. In einer Variante werden die oben erwähnten Pulsselektoren als Schalter verwendet. Zum Umschalten zwischen Femtosekundenlaseroszillator 11 und Picosekundenlaseroszillator 120 können den Laseroszillatoren zudem auch Shutters 151 zur Unterdrückung von Laserpulsen nachgeschaltet werden. Die Femtosekundenlaserpulse FP und Picosekundenlaserpulse PP werden zur Zuführung zum Lichtprojektor 16 über Umlenkspiegel 153 und strahlvereinigende Elemente 154, z.B. teildurchlässige Spiegel, auf eine gemeinsame Projektionsachse geleitet.

In der Ausführungsvariante gemäss der Figur 3 erfolgt die Selektion von Femtosekundenlaserpulsen FP oder Picosekundenlaserpulsen PP aufgrund unterschiedlicher Polarisation. Wie in Figur 3 ersichtlich ist, sind dem Femtosekundenlaseroszillator 11 und/oder dem Picosekundenlaseroszillator 120 jeweils ein Polarisationsmodulator 112, 122 nachgeschaltet, welche die Femtosekundenlaserpulse FP respektive Picosekundenlaserpulse PP unterschiedlich polarisiert weiterleiten, beispielsweise mit gegenseitig normaler Polarisation. Dazu sind die Polarisationsmodulatoren 112, 122 eingerichtet, die Polarisation der Laserpulse unterschiedlich zu drehen oder die Laserpulse verschieden zu polarisieren. Die unterschiedlich polarisierten Femtosekundenlaserpulse FP und Picosekundenlaserpulse PP werden über Umlenkspiegel 153 einem steuerbaren Polasierungsstrahlteiler 150 zugeführt der abhängig von seiner Ansteuerung und der Polarisation der Laserpulse wahlweise die Femtosekundenlaserpulse FP oder die Picosekundenlaserpulse PP an den Lichtprojektor 16 zur Projektion auf oder ins Augengewebe durchleitet. Nicht durchgeleitete Laserpulse werden beispielsweise einem optionalen Beam-Dump-Modul 156 zugeführt. In einer vereinfachten und bevorzugten Ausführung sind der Femtosekundenlaseroszillator 11 und/oder der Picosekundenlaseroszillator 120 eingerichtet die Femtosekundenlaserpulse FP respektive Picosekundenlaserpulse PP unterschiedlich polarisiert abzugeben, so dass sich nachgeschaltete Polarisationsmodulatoren 112, 122 erübrigen.

In der Variante gemäss Figur 4 erfolgt die Selektion von Femtosekundenlaserpulsen FP oder Picosekundenlaserpulsen PP mittels eines steuerbaren Drehspiegels 155 oder mittels einer Kombination von Strahlteiler 152 und Shutters 151. In der einen Variante werden die Femtosekundenlaserpulse FP durch den Drehspiegel 155 wahlweise direkt dem Lichtprojektor 16 zugeführt oder zur Erzeugung von Picosekundenlaserpulsen PP über weitere Umlenkspiegel 153 durch den im Picosekundenlasermodul 12 angeordneten Pulsstrecker 123 zum Lichtprojektor 16 geleitet. In der anderen Variante werden die Femtosekundenlaserpulse FP über den Strahlteiler 152 via jeweils einen steuerbaren Shutter 151 sowohl auf dem direkten Weg dem Lichtprojektor 16 zugeführt oder über Umlenkspiegel 153 durch den im Picosekundenlasermodul 12 angeordneten Pulsstrecker 123, den optionalen optischen Verstärker 124 und den optionalen Kompressor 125 zum Lichtprojektor 16 geleitet. Durch entsprechendes Öffnen oder Schliessen der Shutters 151 können dem Lichtprojektor 16 somit Femtosekundenlaserpulse FP und/oder Picosekundenlaserpulse PP zugeführt werden.

In der Variante gemäss Figur 5 erfolgt die Selektion von Femtosekundenlaserpulsen FP oder Picosekundenlaserpulsen PP durch entsprechendes Zuschalten oder Wegschalten des Verstimmglieds 110.

In den Figuren 1 bis 5 bezieht sich das Bezugszeichen 13 auf ein elektronisches Steuermodul zur Steuerung der ophthalmologischen Vorrichtung 1. Das Steuermodul 13 ist vorzugsweise als programmiertes Softwaremodul ausgeführt, das einen oder mehrere Prozessoren der ophthalmologischen Vorrichtung 1 zur Ausführung von nachfolgend beschriebenen Funktionen steuert und in einem fest oder entfernbar mit den Prozessoren verbundenen, greifbaren, beispielsweise nicht-transienten, computerlesbaren Speichermedium gespeichert ist. Der Fachmann wird verstehen, dass das Steuermodul oder zumindest einige seiner Funktionen in alternativen Ausführungsvarianten teilweise oder vollständig mit hardwarebasierten Komponenten ausführbar sind.

Abhängig von Benutzerinstruktionen setzt das Steuermodul 13 die ophthalmologische Vorrichtung 1 in einen von mehreren nachfolgend beschriebenen Betriebsmodi und steuert die Komponenten und funktionalen Module der ophthalmologischen Vorrichtung 1 entsprechend dem gesetzten Betriebsmodus.

In einem ersten Betriebsmodus, zum Schneiden des Augengewebes, werden die Femtosekundenlaserpulse FP dem Lichtprojektor 16 zur fokussierten Projektion ins Augengewebe, insbesondere in die Cornea 21, zugeführt. Abhängig von der Ausführungsvariante wird dazu der Femtosekundenlaseroszillator 11 bei ausgeschaltetem Picosekundenlaseroszillator 120 eingeschaltet; der Polasierungsstrahlteiler 150 zum Durchlassen der Polarisation der Femtosekundenlaserpulse FP und Nichtdurchlassen der Polarisation der Picosekundenlaserpulse PP eingestellt und, abhängig von der Ausführungsvariante, gegebenenfalls die Polarisation der Femtosekundenlaserpulse FP für den Durchlass und die Polarisation der Picosekundenlaserpulse PP für den Nichtdurchlass durch den Polasierungsstrahlteiler 150 verändert; die Femtosekundenlaserpulse FP vom Drehspiegel 155 respektive dem Strahlteiler 152 und Shutters 151 direkt dem Lichtprojektor 16 zugeführt; oder das Verstimmglied 110 im Femtosekundenlaseroszillator 11 ausrespektive weggeschaltet.

In einem zweiten Betriebsmodus, zum Fragmentieren des Augengewebes, werden die Picosekundenlaserpulse PP dem Lichtprojektor 16 zur fokussierten Projektion ins Augengewebe, insbesondere in die Augenlinse 22, zugeführt. Abhängig von der Ausführungsvariante wird dazu der Picosekundenlaseroszillator 12 bei ausgeschaltetem Femtosekundenlaseroszillator 11 eingeschaltet; der Polasierungsstrahlteiler 150 zum Durchlassen der Polarisation der Picosekundenlaserpulse PP und Nichtdurchlassen der Polarisation der Femtosekundenlaserpulse FP eingestellt und, abhängig von der Ausführungsvariante, gegebenenfalls die Polarisation der Picosekundenlaserpulse PP für den Durchlass und die Polarisation der Femtosekundenlaserpulse FP für den Nichtdurchlass durch den Polasierungsstrahlteiler 150 verändert; die Femtosekundenlaserpulse FP vom Drehspiegel 155 respektive dem Strahlteiler 152 und Shutters 151 zur Erzeugung von Picosekundenlaserpulsen PP durch den im Picosekundenlasermodul 12 angeordneten Pulsstrecker 123 zum Lichtprojektor 16 geleitet; oder das Verstimmglied 110 im Femtosekundenlaseroszillator 11 zur Erzeugung von Picosekundenlaserpulsen PP einrespektive zugeschaltet.

In einem dritten und vierten Betriebsmodus werden die Picosekundenlaserpulse PP und Femtosekundenlaserpulse FP dem Lichtprojektor 16 zur Bearbeitung des Augengewebes in Kombination zugeführt. Dabei wird dem Lichtprojektor 16 eine Sequenz von jeweils paarweise kombinierten Laserpulsen zugeführt. In der kombinierten Zuführung von Laserpulsen werden dem Lichtprojektor 16 jeweils ein Picosekundenlaserpuls PP und ein Femtosekundenlaserpuls FP mit zeitlich versetztem Pulsbeginn zur fokussierten Projektion an den selben Arbeitspunkt zugeführt, entweder ein Femtosekundenlaserpuls FP gefolgt von einem Picosekundenlaserpuls PP oder ein Picosekundenlaserpuls PP gefolgt von einem Femtosekundenlaserpuls FP. In einer Variante überlappen sich die direkt aufeinander folgenden Laserpulse einer Pulskombination zeitlich. Abhängig von der Ausführungsvariante werden zur Erzeugung einer Laserpulskombination jeweils der Picosekundenlaseroszillator 120 und der Femtosekundenlaseroszillator 11 mit entsprechend zeitlich versetzter Pulserzeugung d.h. entsprechender Phasenverschiebung zwischen Pulsbeginn betrieben, z.B. in den Varianten gemäss den Figuren 2 und 3, oder bei synchronisierter Pulserzeugung, jeweils ohne Phasenverschiebung zwischen Femtosekundenlaserpuls FP und Picosekundenlaserpuls PP, mit einem entsprechenden zuschaltbaren optischen Verzögerungselement 126 ausgeführt und betrieben, z.B. in den Varianten gemäss Figur 4. Das Verzögerungselement 126 ist vorzugsweise im Strahlengang vor dem optischen Verstärker 124 angeordnet, d.h. dem optischen Verstärker 124 vorgeschaltet.

Durch die Kombination von zwei nacheinander folgenden und sich beispielsweise teilweise überlappenden Laserpulsen im dritten und vierten Betriebsmodus können die Pulsenergie und/oder die Pulsintensitäten der beiden Pulse geeignet kombiniert werden. Beispielsweise kann mit einem vorangehenden Femtosekundenlaserpuls FP ein initiale Zündung bewirkt und mit dem nachfolgenden Picosekundenlaserpuls PP zusätzliche Energie in die gezündete Wechselwirkungszone gepumpt werden. Im Kombinationsbetrieb ist das Augengewebe auch mit Nanosekundenlaserpulsen im oberen Nanobereich z.B. im Bereich von 1ns bis 10ns bearbeitbar insbesondere für die Fragmentierung der Linse. Wenn die Vorrichtung 1 eingerichtet ist, "saubere" Nanosekundenlaserpulse ohne Spikes und/oder Amplitudenschwankungen zu erzeugen, dann ist sie auch zum Schneiden einsetzbar. Zur Erzeugung der Nanosekundenlaserpulse werden die Femtosekundenlaserpulse FP respektive Picosekundenlaserpulse PP entsprechend gestreckt respektive ein entsprechendes Verstimmglied 110 zugeschaltet. Der Fachmann wird zudem verstehen, dass die ophthalmologische Vorrichtung 1 in weiteren Ausführungsvarianten zudem mit wellenlängenkonvertierenden Elementen ausgestattet ist.

Das Steuermodul 13 ist überdies eingerichtet, die Pulsrate abhängig vom gewählten Betriebsmodus und/oder basierend auf Benutzerinstruktionen zu setzen und den Pulsselektor 111 entsprechend anzusteuern.

Das Steuermodul 13 umfasst zudem ein gemeinsames elektronisches Lasersteuermodul 130 zum Steuern des Femtosekundenlaseroszillators 11 und des Picosekundenlasermoduls 12, insbesondere des Picosekundenlaseroszillators 120. Das Lasersteuermodul 130 ist eingerichtet die Pulsenergie, Pulsbreite, maximale Pulsintensität (je nach Pulsform ergeben sich unterschiedliche Werte der Pulsintensität, bei sonst gleicher Pulsbreite und Pulsenergie), Pulsrate, Wellenlänge, Fokusgrösse, und/oder mittlere Laserleistung der Laseroszillatoren zu steuern.

Abschliessend soll festgehalten werden, dass die ophthalmologische Vorrichtung 1 überdies nicht dargestellte strahlablenkende Mittel zum örtlichen Abtasten (Scannen) des zu bearbeitenden Augengewebes mit den Femto- FP und/oder Picosekundenlaserpulse PP umfasst.

## Patentansprüche

1. Ophthalmologische Vorrichtung (1) zur Bearbeitung von Augengewebe, umfassend:
einen Laserpulsgenerator (10) zum Erzeugen von Laserpulsen (P),
einen Lichtprojektor (16) zur fokussierten Projektion der Laserpulse (P) auf oder in das Augengewebe,
wobei der Laserpulsgenerator (10) einen Femtosekundenlaseroszillator (11) zur Erzeugung von Femtosekundenlaserpulsen (FP) umfasst, **gekennzeichnet durch**
ein Picosekundenlasermodul (12) zur Erzeugung von Picosekundenlaserpulsen (PP), wobei dem Lichtprojektor (16) zur Bearbeitung des Augengewebes wahlweise Femtosekundenlaserpulse (FP) und/oder Picosekundenlaserpulse (PP) zuführbar sind.

2. Vorrichtung (1) nach Anspruch 1, **gekennzeichnet durch** ein Steuermodul (13), das eingerichtet ist, die Vorrichtung (1) wahlweise in mindestens einen der nachfolgenden Betriebsmodi zu setzen und zu steuern:
- ein erster Betriebsmodus, in welchem vom Laserpulsgenerator (10) dem Lichtprojektor (16) Femtosekundenlaserpulse (FP) zum Schneiden des Augengewebes zugeführt werden,
- ein zweiter Betriebsmodus, in welchem vom Laserpulsgenerator (10) dem Lichtprojektor (16) Picosekundenlaserpulse (PP) zum Fragmentieren des Augengewebes zugeführt werden,
- ein dritter Betriebsmodus, in welchem vom Laserpulsgenerator (10) dem Lichtprojektor (16) zur Bearbeitung des Augengewebes jeweils mit zeitlich versetztem Pulsbeginn ein Picosekundenlaserpuls (PP) gefolgt von einem Femtosekundenlaserpuls (FP) zugeführt werden, und
- ein vierter Betriebsmodus, in welchem vom Laserpulsgenerator (10) dem Lichtprojektor (16) zur Bearbeitung des Augengewebes jeweils mit zeitlich versetztem Pulsbeginn ein Femtosekundenlaserpuls (FP) gefolgt von einem Picosekundenlaserpuls (PP) zugeführt werden.

3. Vorrichtung (1) nach einem der Ansprüche 1 oder 2, **dadurch gekennzeichnet, dass** das Picosekundenlasermodul (12) einen Picosekundenlaseroszillator (120) zum Erzeugen der Picosekundenlaserpulse (PP) umfasst.

4. Vorrichtung (1) nach einem der Ansprüche 1 oder 2, **dadurch gekennzeichnet, dass** das Picosekundenlasermodul (12) einen Pulsstrecker (123) umfasst, welcher eingerichtet ist, Femtosekundenlaserpulse (FP) vom Femtosekundenlaseroszillator (11) in Picosekundenlaserpulse (PP) zu strecken.

5. Vorrichtung (1) nach einem der Ansprüche 1 oder 2, **dadurch gekennzeichnet, dass** das Picosekundenlasermodul (12) ein im Femtosekundenlaseroszillator (11) angeordnetes einschaltbares Verstimmglied (110) umfasst, welches eingerichtet ist, den Femtosekundenlaseroszillator (11) im eingeschalteten Zustand so zu verändern, dass der Femtosekundenlaseroszillator (11) als Picosekundenlaseroszillator betreibbar ist.

6. Vorrichtung (1) nach einem der Ansprüche 1 bis 4, **dadurch gekennzeichnet, dass** eine Polarisationsmodulation vorgesehen ist, derart, dass die Femtosekundenlaserpulse (FP) und die Picosekundenlaserpulse (PP) unterschiedlich polarisiert sind, und dass die Vorrichtung (1) einen Polasierungsstrahlteiler (150) umfasst, um dem Lichtprojektor (16) wahlweise Femtosekundenlaserpulse (FP) oder Picosekundenlaserpulse (PP) zuzuführen.

7. Vorrichtung (1) nach einem der Ansprüche 1 bis 6, **dadurch gekennzeichnet, dass** zum wahlweisen Zuführen von Femtosekundenlaserpulsen (FP) respektive Picosekundenlaserpulsen (PP) zum Lichtprojektor (16) mindestens eines aus den folgenden Elementen vorgesehen ist: ein steuerbarer Shutter (151), ein steuerbarer Drehspiegel, ein elektromechanischer Schalter und ein elektronischer Schalter.

8. Vorrichtung (1) nach einem der Ansprüche 1 bis 7, **dadurch gekennzeichnet, dass** die Femtosekundenlaserpulse (FP) zum Schneiden des Augengewebes fokussiert in die Cornea (21) projizierbar sind, und dass die Picosekundenlaserpulse (PP) zum Fragmentieren des Augengewebes fokussiert in die Augenlinse (22) projizierbar sind.

9. Vorrichtung (1) nach einem der Ansprüche 1 bis 8, **dadurch gekennzeichnet, dass** der Laserpulsgenerator (10) mit dem Femtosekundenlaseroszillator (11) und dem Picosekundenlasermodul (12) in einem gemeinsamen Gehäuse angeordnet ist, und dass die ophthalmologische Vorrichtung (1) zum Thermalisieren des Laserpulsgenerators (10) eine mit dem Gehäuse verbundene Kühlvorrichtung (14) umfasst.

10. Vorrichtung (1) nach einem der Ansprüche 1 bis 9, **gekennzeichnet durch** ein mit dem Femtosekundenlaseroszillator (11) und dem Picosekundenlasermodul (12) verbundenes gemeinsames elektronisches Lasersteuermodul (130) zum Steuern des Femtosekundenlaseroszillators (11) und des Picosekundenlasermoduls (12), und ein mit dem Femtosekundenlaseroszillator (11) und dem Picosekundenlasermodul (12) verbundenes gemeinsames Speisegerät (17) zur elektrischen Speisung des Femtosekundenlaseroszillators (11) und des Picosekundenlasermoduls (12).

11. Vorrichtung (1) nach einem der Ansprüche 1 bis 10, **dadurch gekennzeichnet, dass** dem Femtosekundenlaseroszillator (11) ein Pulsselektor (111) zum Erzeugen einer bestimmten Pulsrate nachgeschaltet ist, und dass die Pulsrate abhängig von einem gewählten Betriebsmodus setzbar ist.

12. Vorrichtung (1) nach einem der Ansprüche 1 bis 11, **gekennzeichnet durch** eine gemeinsame Pumpquelle für den Femtosekundenlaseroszillator (11) und das Picosekundenlasermodul (12).

13. Vorrichtung (1) nach einem der Ansprüche 1 bis 12, **dadurch gekennzeichnet, dass** das Picosekundenlasermodul (12) einen optischen Verstärker (124) umfasst.

14. Vorrichtung (1) nach einem der Ansprüche 1 bis 13, **dadurch gekennzeichnet, dass** das Picosekundenlasermodul (12) einen optischen Kompressor (125) zum Komprimieren der Pulslänge der Picosekundenlaserpulse (PP) umfasst.

15. Vorrichtung (1) nach einem der Ansprüche 1 bis 14, **dadurch gekennzeichnet, dass** der Laserpulsgenerator (10) eingerichtet ist, Femtosekundenlaserpulse (FP) mit einer Pulsrate im Bereich von 1-100MHz und Picosekundenlaserpulse (PP) mit einer Pulsrate im Bereich von 1 KHz-1 MHz zu erzeugen.
